# EUROPEAN PATENT APPLICATION

(11) **EP 2 042 591 A1**
(43) Date of publication of application: **01.04.2009**
(21) Application number: 07768152.6
(22) Date of filing: 04.07.2007
(51) Int. Cl.: C12N 5/00

(54) **ERYTHROPOIETIN-PRODUCING ORGANOID PRECURSOR, METHOD OF CONSTRUCTING THE SAME AND METHOD OF TREATING ERYTHROPOIETIN-RELATED DISEASE**

(30) Priority: 04.07.2006 JP 2006185004
(71) Applicant: Stemcell Institute Inc., Tokyo 105-0004 (JP)
(72) Inventor: YOKOO, Takashi, Tokyo 105-8461 (JP); OKABE, Masataka, Tokyo 105-8461 (JP); HOSOYA, Tatsuo, Tokyo 105-8461 (JP)
(74) Representative: De Clercq, Ann G. Y.
(86) International application number: PCT/JP2007/063400
(87) International publication number: WO 2008/004598

(57) **Abstract**

The subject of the present invention is to provide a means to producing an erythropoietin-producing organoid using mesenchymal stem cell derived from a mammal. It is a method for producing erythropoietin-producing organoid (organ-like structure) precursor, comprising the step of transplanting mesenchymal stem cell derived from a mammal into an embryo within a pregnant mammalian host or an embryo separated from a pregnant mammalian host to thereby induce the differentiation of the mesenchymal stem cell, in particular, a site to which the mesenchymal stem cell is to be transplanted is a nephrogenic site of the embryo, and a timing of transplantation corresponds to the stage in which a immune system of the host is still immunologically tolerant.

## Description

### Technical Field

The present invention is to provide erythropoietin-producing organoid precursor, the producing method thereof and a method for treating erythropoietin-related disorder.
The present invention claims priority from Japanese Patent Application No. 2006-185004, the content of which is incorporated herein by reference.

### Background Art

Kidney is a main organ to produce erythropoietin, and the decreased erythropoietin production associated with renal failure causes renal anemia complications. Currently, this renal anemia has been treated by the administration of a purified recombinant protein. The administration of recombinant protein from 1,500 to 3,000 units is required for a patient with end-stage renal failure on each dialysis, and the agents are extremely expensive (currently, 5, 697 yen for 3,000 units). The number of dialysis patients has currently exceeded 250,000, and it is expected to further explosively increase in the future with the aging of population and the increase in diabetes, which may impose an enormous financial burden. Further, it has been reported that the administration of the recombinant protein may generate anti-erythropoietin antibody, resulting in causing pure red cell aplasia, and therefore an alternative therapy to the recombinant protein has been required.

In conventional therapy, there is reported a method of causing cells to forcibly express erythropoietin using viruses or the like, and transplanting the cells intradermally, but the expression of erythropoietin can occur transiently only within living cells and the forced expression may continue even after the correction of anemia, which leaves a fear for polycythemia. Therefore, there is a need for a regulatory mechanism which allows cells to secrete it in a necessary amount when needed (in other words, at the time of anemia and the like), and further it is essential for a clinical application to be able to supply erythropoietin stably for a long time by in vivo engraftment.

Meanwhile, currently, a method is drawing attention as a next-generation therapy, which can differentiate autologous bone marrow cells or ES cells into cells having a particular function. For example, a method of differential induction into pancreatic β cells having insulin secretory ability has been developed. However, up until now, the differential induction of bone marrow or ES cells into erythropoietin-producing cells has not been developed.

Further, organ transplantation technology along with the progress of transplantation therapy has brought a hope for the complete recovery from organ failure by transplantation, which has hardly been ameliorated until now. However, donors are chronically insufficient worldwide, and even after the successful transplantation, the long-term dosing of immunosuppressants to avoid rejection is required, which entails a long-lasting fight against accompanying side effects (nonpatent document No. 1).

Therefore, one goal of ultimate therapeutic strategy is to produce erythropoietin-producing organoid precursor from autologous tissue stem cell and to transplant the in vitro-derived organoid precursor back into the individual donor again as an autologous graft.
Further, it has recently been revealed that human mesenchymal stem cells found in adult bone marrow may maintain their plasticity depending on their microenvironment and differentiate into several different types of cells (nonpatent document No. 2). In comparison with embryonic stem cell (ES cell), human mesenchymal stem cell can be separated from autologous bone marrow, and can be applied to therapy without raising serious ethical problems or involving immunological results (nonpatent document No. 3).
[Nonpatent document No. 1] Transplantation 77, S41-S43 (2004)
[Nonpatent document No. 2] Science 276, 71-74 (1997)
[Nonpatent document No. 3] Birth Defects Res. 69, 250-256 (2003)
[Nonpatent document No. 4] J. Neurosci. Res. 60, 511-519 (2000)
[Nonpatent document No. 5] Blood 98, 57-64 (2001)
[Nonpatent document No. 6] J. Am. Soc. Nephrol. 11, 2330-2337 (2001)
[Nonpatent document No. 7] Methods 24, 35-42 (2001)
[Nonpatent document No. 8] J. Clin. Invest. 105, 868-873 (2000)

### Disclosure of the Invention

### Problem to be solved by the Invention

To solve the drawbacks described above, the subject of the present invention is to provide erythropoietin-producing organoid which (1) can be obtained from autologous mesenchymal stem cell, capable of avoiding immune rejection, (2) can sustain the long-term expression of erythropoietin by in vivo engraftment into the individual patient, and further (3) can regulate the amount of expression of erythropoietin, and a method for treating erythropoietin-related disorder.

### Means for Solving the Problem

The present inventors have found that erythropoietin-producing organoid precursor can be produced by transplanting human mesenchymal stem cell onto the nephrogenic site of a growing embryo followed by performing each culturing step, and thus have completed one of the present inventions. Further, the present inventors have completed a method for treating erythropoietin-related disorder by preparing erythropoietin-producing organoid through transplantation of the erythropoietin-producing organoid precursor into the greater omentum of a recipient (patient) with erythropoietin-related disorder.

Therefore, the present invention includes:
1. A method for producing erythropoietin-producing organoid (organ-like structure) precursor, comprising the step of transplanting isolated mesenchymal stem cell derived from a mammal into an embryo within a pregnant mammalian host or an embryo separated from a pregnant mammalian host to thereby induce the differentiation of the mesenchymal stem cell, wherein a site to which the mesenchymal stem cell is to be transplanted is a nephrogenic site of the embryo, and a timing of transplantation corresponds to the stage in which a immune system of the host is still immunologically tolerant.
2. The method for producing erythropoietin-producing organoid precursor according to the preceding aspect 1, further comprising in vitro whole-embryo culture.
3. The method for producing erythropoietin-producing organoid precursor according to the preceding aspect 2, further comprising in vitro organ culture.
4. The method for producing erythropoietin-producing organoid precursor according to any one of the preceding aspects from 1 to 3, wherein the erythropoietin-producing organoid precursor has an ability to adjust erythropoietin production by transplantation into greater omentum of a mammal.
5. Erythropoietin-producing organoid precursor obtained by the method for producing erythropoietin-producing organoid precursor according to any one of the preceding aspects 1 to 4.
6. Erythropoietin-producing organoid precursor obtained by the following steps:
   (1) transplanting isolated mesenchymal stem cell derived from a mammal onto a nephrogenic site of an embryo separated from a pregnant mammalian host at a timing when the immune system of the host is still in the immunological tolerance stage, to thereby induce the differentiation of the mesenchymal stem cell,
   (2) performing in vitro whole-embryo culture, and
   (3) performing in vitro organ culture.
7. The erythropoietin-producing organoid precursor according to either of the preceding aspects 5 or 6, wherein the erythropoietin-producing organoid precursor has an ability to adjust erythropoietin production by transplantation into greater omentum of a mammal.
8. A kit for producing erythropoietin-producing organoid precursor containing at least the following:
   (1) isolated mesenchymal stem cell derived from a mammal,
   (2) a pregnant mammal or an embryo separated from a pregnant mammal,
   (3) a reagent for performing whole-embryo culture,
   (4) a reagent for performing organ culture, and
   (5) an instruction for producing erythropoietin-producing organoid precursor.
9. A method for treating erythropoietin-related disorder, comprising the step of transplanting the erythropoietin-producing organoid precursor according to any one of the preceding aspects 5 to 7 into greater omentum of a mammal.
10. The method according to the preceding aspect 9, wherein the erythropoietin-producing organoid precursor is derived from mesenchymal stem cell in a recipient (patient) with erythropoietin-related disorder.

### (Effects of Invention)

The present invention has provided erythropoietin-producing organoid necessary for the treatment of erythropoietin-related disorder and the production method thereof. That is to say, the method is transplanting erythropoietin-producing organoid precursor into the greater omentum of a recipient with erythropoietin-related disorder, wherein the erythropoietin-producing organoid precursor can sustain the long-term expression of erythropoietin and regulate the amount of expression of erythropoietin, that is different from conventional erythropoietin-producing cells which can produce erythropoietin transiently and/or produce it in more than the required amount.

### Brief description of the drawings

Figure 1 shows the emergence of erythropoietin-producing organoid transplanted into greater omentum.
Figure 2 shows the tissue analysis of erythropoietin-producing organoid transplanted in greater omentum (2nd week).
Figure 3 shows that blood vessels from the recipient are constructed in erythropoietin-producing organoid.
Figure 4 shows an electron micrograph of erythropoietin-producing organoid transplanted into greater omentum. It shows that erythrocytes can be found in glomerular tuft, which suggesting vascular integration with the blood flow of the recipient.
Figure 5 shows the results of the expression of human erythropoietin mRNA using RT-PCR.
Figure 6 shows the results of double staining using anti-erythropoietin antibody and antihuman β₂ microglobulin antibody.
Figure 7 shows the measurement results of erythropoietin concentration in the serum of each rat before and after anemia.
Figure 8 shows the result of the effect of erythropoietin-producing organoid on correcting anemia.
Figure 9 shows the results confirming the possibility of controlled release of GDNF using a temperature-sensitive polymer.
Figure 10 shows the results in that a part derived from human mesenchymal stem cell is enlarged by controlled release of GDNF by polymer.

### Description of Preferred Embodiments

The present invention is a method for producing erythropoietin-producing organoid (organ-like structure) precursor, comprising the step of transplanting isolated mesenchymal stem cell derived from a mammal into an embryo within a pregnant mammalian host or an embryo separated from a pregnant mammalian host to thereby induce the differentiation of the mesenchymal stem cell, in particular wherein a site to which the mesenchymal stem cell is to be transplanted is a nephrogenic site of the embryo, and a timing of transplantation corresponds to the stage in which a immune system of the host is still immunologically tolerant.

The term "erythropoietin-producing organoid (organ-like structure)" herein is not a single cell but a cell structure, and has an erythropoietin-producing ability, which is originally a kidney function. Meanwhile, each cell of erythropoietin-producing organoid contacts with each other three-dimensionally except the surface, and adjacent cells share information through a variety of intercellular junctions. And, each cell not only produces erythropoietin alone but also has an ability to adjust the production thereof.
Meanwhile, the term "precursor" herein means a state, though having factors necessary for functions to produce erythropoietin and to adjust the production thereof, which does not exert those functions by an environmental factor. When erythropoietin-producing organoid precursor is transplanted into the greater omentum of, preferably, a mammal, in particular human, it is changed into erythropoietin-producing organoid. That is to say, the erythropoietin-producing organoid precursor exhibits erythropoietin-producing function and erythropoietin production-adjusting function when present in an environment in greater omentum of a mammal, in particular human. However, the erythropoietin-producing organoid precursor can be changed into erythropoietin-producing organoid under a culture condition which is the same environmental state as that of the greater omentum of a mammal, in particular human.

Now, "the erythropoietin production-adjusting function" herein means to produce more amount of erythropoietin (necessary for correcting anemia) when more erythropoietin is needed in a recipient (patient) (for example by anemia) than that needed in a normal condition. However, when the recipient (patient) is in a normal condition (for example, at a time when anemia has been corrected), erythropoietin may be produced in the required amount to maintain the normal inner body condition.
That is to say, the erythropoietin-producing organoid of the present invention is quite different from the conventional erythropoietin-producing cells in that it can produce the amount of erythropoietin required by the recipient (patient), and further, in that it can keep producing erythropoietin for a long time by taking necessary nutrition from blood vessels within greater omentum.

The term "erythropoietin-related disorder" herein means diseases related to the decrease in the amount of erythropoietin production, including anemia associated with renal failure, diabetes mellitus, ulcers, cancers, infections, dialysis, surgeries and chemotherapies. In particular, in the case of anemia caused by chronic renal failure, there remains a big problem that erythropoietin cannot be produced by the progressive destruction of parenchyma renis and liver functions, resulting in limiting the increase in erythropoietin concentration in the circulatory system. The patient (recipient) refers to all mammals including human. For example, it refers to human and pets such as monkey, cattle, sheep, pig, goat, horse (in particular, race horse), dog, cat, rabbit, hamster, guinea pig, rat, and mouse.

As a suitable example of mammalian hosts useful in the present invention, pig and the like may be mentioned, and the other suitable animals include genetically modified transgenic, knockout, knock-in pigs and the like. Besides them, ungulates such as cattle, sheep, pig, goat and horse may be exemplified. Further, rats or genetically modified animals from ungulates described above, in particular, the transgenic animals of them may be exemplified.

As mesenchymal stem cells derived from a mammal, those derived from a recipient are preferably used. For example, when the recipient is human, they may be isolated from the bone marrow, blood flow or umbilical cord blood of the human who will receive transplantation of erythropoietin-producing organoid precursor. Especially given the case of adult patients with erythropoietin-related disorder, particularly renal failure etc., human mesenchymal stem cells from autologous bone marrow are preferred.
The preparative isolation is performed by a general surgical procedure. The isolated cells may be cultured under the optimal condition, and should not be beyond 2 to 5 cell passages. In order to keep culturing while not allowing human mesenchymal stem cells to be transformed, a culture media kit specialized for human mesenchymal stem cells and supplied by Cambrex BioScience may be used.
If required, the desired gene may be introduced into human mesenchymal stem cell by adenoviral and/or retroviral techniques etc. For example, genes may be introduced so as to express GDNF (Glial cell line-derived neurotrophic factor). It was confirmed that this introduction could increase the formation rate of erythropoietin-producing organoid precursor derived from the injected stem cells by from 5.0 ± 4.2% to 29.8 ± 9.2%.
Further, other than the adenoviral and/or retroviral techniques described above, GDNF can be released in a controlled manner by mixing GDNF-containing polymer, in particular temperature-sensitive Mebiol Gel with mesenchymal stem cells derived from a mammal before injection.

Then, the prepared mesenchymal stem cell derived from a mammal may be transplanted into an embryo within a pregnant mammalian host. Namely, mesenchymal stem cell derived from a mammal can be directly transplanted into an embryo within a living body to form erythropoietin-producing organoid precursor in utero. Typical surgical techniques can be used for transplantation. For example, for a system using pig as a host, mesenchymal stem cell derived from a mammal may be injected by echo guidance into an embryo in utero using a micropipette, etc. The cellular quantity of 0.5 X 10³ to 1.0 X 10³ is sufficient for the transplantation. Namely, the mesenchymal stem cells derived from a mammal are directly transplanted by a transuterine approach into an embryo within the live body of a large pregnant mammal such as a pig, and left to grow inside the live body into erythropoietin-producing organoid precursor. In addition, after that, the process of "whole-embryo culture" or "organ culture" as shown below can be added, but those steps are not always required, because erythropoietin-producing organoid precursor may have been sufficiently grown. In addition, human mesenchymal stem cells are preferably used.

Further, preferably, the prepared mesenchymal stem cell derived from a mammal can be transplanted into an embryo separated from a pregnant mammalian host (uterus), then matured in vitro in the embryo until the completion of the initial stage of organogenesis (erythropoietin-producing organoid) using whole-embryo culture, and then further cultured into erythropoietin-producing organoid precursor by organ culture. Further, erythropoietin-producing organoid can be obtained by transplantation of the erythropoietin-producing organoid precursor into greater omentum of a mammal. In addition, human mesenchymal stem cells are preferably used.

In the present invention, it has been found that mesenchymal stem cell derived from a mammal can be differentiated into erythropoietin-producing organoid by the processes described above, and this new erythropoietin-producing organoid has a function to adjust erythropoietin production.

The timing of transplantation into an embryo is selective. In experiments using rats, rats in E (stage embryo) from 9 to 12 (day-old), in particular E from 10 to 12, more preferably E from 10 to 11.5 and most preferably E11.5 were used. In a large mammal such as pig, similar stage embryos can preferably be used. However, by selecting appropriate conditions, an earlier or later stage can also be selected. In any case, it is important that the cell should be transplanted into the embryo at least at a time when the host is still at an immunologically tolerant stage.

The present invention is characterized by the selection of transplantation site into an embryo. Namely, the transplantation site of mesenchymal stem cell derived from a mammal into an embryo is a site corresponding to the nephrogenic site of the host. Therefore, although transplantation should be conducted at timing when the site can be confirmed to correspond to kidney, it is preferable that bud cells in the kidney are in a sprouting state prior to starting development. For example, transplanting mesenchymal stem cell derived from a mammal onto the site around ureteric budding site, in particular onto the site between the somite and the lateral plate allow erythropoietin-producing organoid to differentiate. Preferably, the cell is transplanted into the metanephros-forming mesoderm. In addition, human mesenchymal stem cells are preferably used.

"Whole-embryo culture" of the present invention is performed when mesenchymal stem cell derived from a mammal is transplanted into the embryos separated from pregnant mammalian host animals (uteri). The outline of whole-embryo culture is that uteri are dissected from mothers, and mesenchymal stem cell derived from a mammal is transplanted into embryos freed from the uterine wall, decidua, and the outer-membrane layer, including Reichert's membrane, and then embryos are cultured in a culture bottle or the like. If the aim of culturing erythropoietin-producing organoid precursor of the present invention can be achieved with "whole-embryo culture," some improvement may be introduced and/or some process may be deleted in the aforementioned culture method. The following is provided to illustrate in more detail but is not limited to this.
Whole embryos were cultured *in vitro* according to a previously described method (non-patent document 7), with several modifications. Using a surgical microscope and the like, uteri were dissected from anaesthetized mothers. The rat embryos which are preferably E9-12, specially E10-12, more preferably E10-11.5, and still more preferably E11.5 are freed from the uterine wall, decidua, and the outer-membrane layer, including Reichert's membrane. The yolk sac and amnion are opened to allow the injection of the mesenchymal stem cell derived from a mammal, but the chorioallantoic placenta is left intact. The embryos confirmed as success in injection of the mesenchymal stem cell derived from a mammal were cultivated in the culture bottles containing 3ml of culture media (glucose, penicillin G, streptomycin, and streptomycin and amphotericin B) comprising of centrifuged rat serum. The culture bottles are allowed to rotate in an incubator (model no. RKI10-0310, Ikemoto, Tokyo). Culture time is preferable 12-60 hours, more preferable 24-48 hours, and still more preferable 48 hours. Furthermore, after a certain culture time, the embryo is preferably assessed in terms of morphology and function, and the organ primordia of erythropoietin-producing organoid precursor are confirmed. After this confirmation, the organ primordia are separated from the embryo to preferably carry out organ culture according to the following method.

The outline of "organ culture" of the present invention is that the above organ primordia are placed on a filter and added DMEM on the dish under them. The dish is incubated in incubator under condition of 5% CO₂. The culture time is preferably 12 to 144 hours, more preferably 18 to 72 hours, still more preferably 24 to 48 hours, and most preferably 24 hours. Accordingly, it is the most effective that the organ primordia are transplanted at the culture time of about 24 hours into the greater omentum. Additionally, the cultivation temperature is preferably 20 to 45°C, more preferably 25 to 40°C and most preferably 37°C. If the aim of culturing the erythropoietin-producing organoid precursor of the present invention can be achieved with "organ culture, some improvement may be introduced and/or some process may be deleted in the aforementioned culture. J. Clin. Invest.105, 868-873(2000) (non Patent document 8) is provided to illustrate in detail but is not to be construed as limiting the scope thereof.

The method of "transplantation of erythropoietin-producing organoid precursor into greater omentum of a mammal" herein can be conducted by a usual surgical approach, for example, by picking up tissues for transplantation with sharp forceps, making a small incision in the surface of adipose tissue of the greater omentum with the ends of the forceps and transplanting the tissue therein. Further, an endoscope can be used for transplanting the tissue into the greater omentum.

Further, in the present invention, a series of manipulations of performing the whole-embryo culture described above for 2 to 60 hours, then the organ culture described above for 12 to 36 hours, and then transplantation into the greater omentum described above is referred to as "improved relay culture."

"A kit for producing erythropoietin-producing organoid precursor" herein comprises at least the following:
(1) isolated mesenchymal stem cell derived from a mammal,
   wherein bone marrow cell derived from a recipient (patient) is preferred,
(2) a pregnant mammal or an embryo separated from a pregnant mammal,
   wherein rat and pig are preferred, but if it is a pregnant mammal or an embryo separated from a pregnant mammal, not limited in particular,
(3) a reagent for performing whole-embryo culture,
   wherein the reagent is necessary for performing "whole-embryo culture" of the present invention and composes a part or the whole of the present application,
(4) a reagent for performing organ culture,
   wherein the reagent is necessary for performing "organ culture" of the present invention and composes a part or the whole of the present application, and
(5) an instruction for producing erythropoietin-producing organoid precursor,
wherein the instruction shows procedures allowing persons skilled in a regenerative medicine field including doctors to produce erythropoietin-producing organoid precursor using (1) to (4) described above.

In "a method for treating erythropoietin-related disorder" herein, erythropoietin-producing organoid precursor is transplanted into the greater omentum of a recipient (patient). The erythropoietin-producing organoid precursor in the greater omentum may be changed into erythropoietin-producing organoid and keep producing erythropoietin for a long time by taking necessary nutrition from blood vessels within greater omentum. In the following examples, erythropoietin-producing organoid in rat greater omentum exerted an effect on correcting anemia of rat models.

Further, the size of erythropoietin-producing organoid precursor to be transplanted does not have to be as large as that of kidney, which is erythropoietin-producing organ, and the size from one-fiftieth to one-tenth of whole kidney may be enough.

In order that the formed erythropoietin-producing organoid may not be contaminated with antigenic substances from the host as foreign substances, the transformation of transplanted cells as follows is effective. Namely, when human mesenchymal stem cells are used, the formed erythropoietin-producing organoid contains a coexistence of human cell derived from human mesenchymal stem cells and the host animal-derived cells. When the erythropoietin-producing organoid is transplanted into human body, the host-derived cells in coexistence are likely to induce an immunological rejection reaction and thus have to be completely removed after the formation of the erythropoietin-producing organoid. In order to solve this problem, the host animal designed to induce controllable programmed cell death is produced and then allowed to form the erythropoietin-producing organoid. The human mesenchymal stem cells are transplanted into the corresponding site of the embryo of the host animal to form erythropoietin-producing organoid, which is then allowed to induce cell death specific to the host cell, thereby to clear completely of the host-derived cells at a step prior to transplantation into human body.

The present invention will be explained below with a system using rat as a representative example of the present invention, but the present invention is not limited thereto.

### Example 1

### Material used and method

### 1) Experimental animal

Wild-type Sprague-Dawley rats were purchased from Sankyo Labo Service Corporation (Tokyo) and used as host animal. The day on which the vaginal plug was observed at midday was designated as 0.5 DPC. The animals were housed in a ventilated cage (under a positive pressured air-flow), bred and kept under a pathogen-free condition. All the experimental procedures had been approved by the Animal Experiment Committee of Jikei University School of Medicine.

### 2) Culture and manipulation of human mesenchymal stem cells

Human mesenchymal stem cells obtained from bone marrow of a healthy volunteer were used. Human mesenchymal stem cells derived from bone marrow, which were confirmed as CD105, CD166-, CD29-, CD44-positive and CD14-, CD34-, CD45-negative, were purchased from Cambrex BioScience Inc. (Walkersville, MD) and were cultured according to the protocol offered by the producer. In order to avoid phenotypical changes, human mesenchymal stem cells were used within five cell passages. A replication-deficient recombinant adenovirus carrying human GDNF cDNA (AxCAhGDNF) was constructed according to the description and purified (nonpatent document No. 4).
Packaging cells (Ψ-crip) which can produce a recombinant retrovirus carrying bacterial LacZ gene (MFG-LacZ) was donated by H. Hamada (Sapporo Medical University). The infections of adenovirus and retrovirus were performed according to the description (nonpatent document Nos. 5 and 6). The cells were labeled with 1,1'-dioctadecyl-3,3,3',3',-tetramethylindocarbocyanine (0.25% DiI (wt/vol)(MolecularProbes)) in 100% dimethylformamide, and injected into the sprouting site of ureteric bud using a micropipette.

### 3) Manipulations of whole-embryo culture and organ culture

Whole embryos were cultured in vitro according to the descried method to which some modifications were made (nonpatent document No. 7). Uteri were removed from the mother body under anesthesia using a stereoscopic microscope. E11.5 (E: stage embryonic day) rat embryos were freed from the uterine wall, decidua, and the outer-membrane layer, including Reichert's membrane. The yolk sac and amnion were opened to allow injection, but the chorioallantoic placenta was left intact. Successfully injected embryos were immediately cultivated in 15-ml culture bottles containing 3 ml of culture media consisting of 100% centrifuged rat serum supplemented with glucose (10mg/ml), penicillin G (100 units/ml), streptomycin (100 micrograms/ml), and amphotericin B (0.25 micrograms/ml). The culture bottles were allowed to rotate in an incubator (model no. RKI10-0310, Ikemoto, Tokyo). Rat embryos were grown ex vivo for a culture period of 48 hours.
Then, the organ primordia after whole-embryo culture was placed on a 0.4 µm-thick Nucleopore filter with a diameter of 12 mm (supplied by Corning-Coster), and 400 µL of 20% PBS DMEM was added to the dish below. Then, the dish containing the organ primordia was subjected to organ culture in a 5% CO₂ incubator at 37°C for 24 hours.

### 4) Transplanting manipulation into greater omentum

It was conducted by picking up erythropoietin-producing organoid precursor obtained in 3) described above with sharp forceps, making a small incision on the surface of adipose tissue of the greater omentum with the ends of the forceps, and then transplanting the erythropoietin-producing organoid precursor therein. At the same time, heminephrectomy was conducted on the rats. In 2 weeks, the rats were subjected to laparotomy.

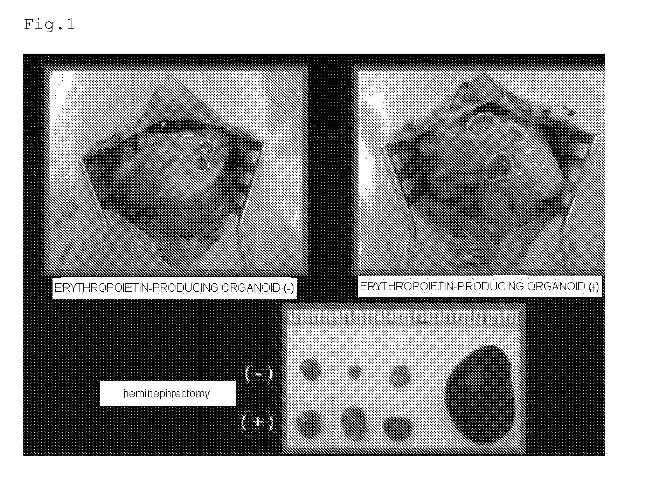

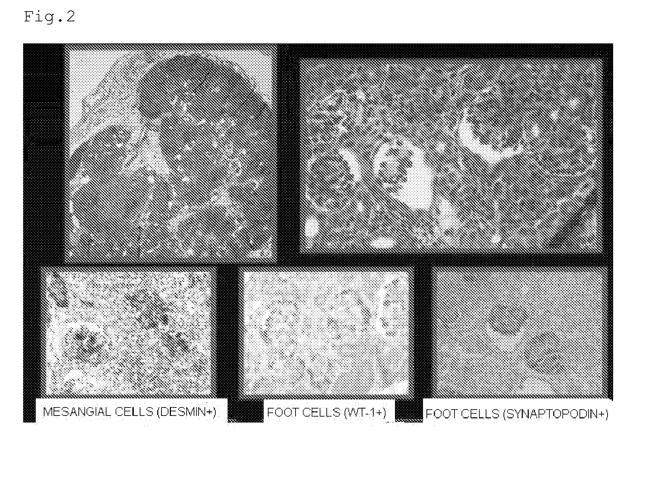

It was confirmed that erythropoietin-producing organoid continued to grow further within the greater omentum and blood vessel system of the greater omentum penetrated into the erythropoietin-producing organoid (Figure 1). This growth showed no decline under renal failure condition (after heminephrectomy), but showed further acceleration to the contrary (Figure 1). The histological analysis of this grown erythropoietin-producing organoid was shown in Figure 2. Erythrocytes were filled in the blood vessels of the erythropoietin-producing organoid, which was not observed before transplantation, and thus the start of blood circulation was histologically demonstrated. Further, glomerular mesangial cells (positive for desmin) and highly differentiated glomerular epithelial cells (cells positive for WT-1 and synaptopodin), which had not been observed before transplanting into greater omentum, were observed.

### 5) Confirmation whether blood vessels were from recipient or not

To confirm that the blood described above is supplied by the blood vessels of rat (recipient), erythropoietin-producing organoid precursor was transplanted into the greater omentum of LacZ rat where the blood vessels of rat were stained blue with LacZ.

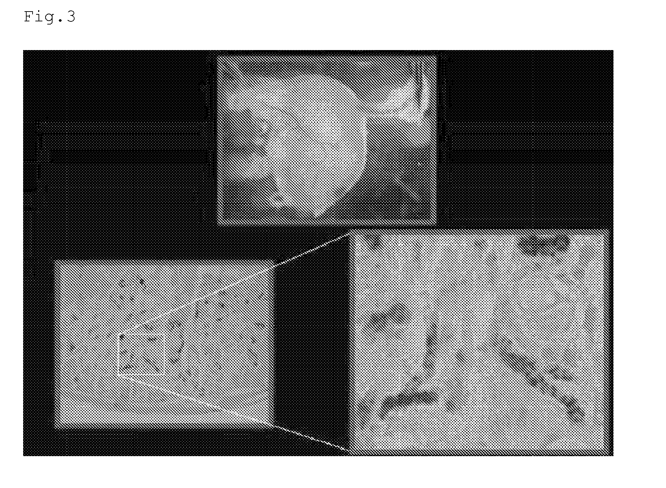

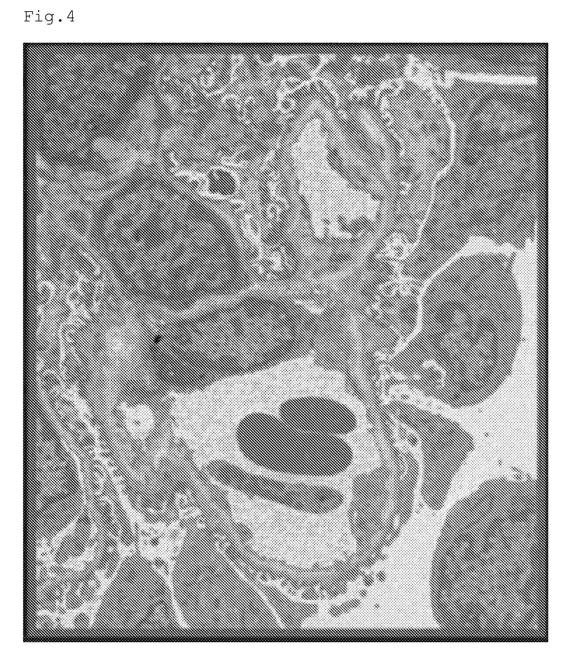

The penetration of the blood vessels of greater omentum into the newly formed erythropoietin-producing organoid was also displayed visually (upper view of Figure 3), and tissue staining with LacZ demonstrated that blood vessels within this erythropoietin-producing organoid was formed with blue cells which was derived from the recipient (lower view of Figure 3). Erythrocytes were also observed in blood vessels within glomerulus with an electron microscope, and further observed were the constructions of the foot processes of highly differentiated glomerular epithelial cells, endothelial and mesangial cells (Figure 4).

### 6) Confirmation of erythropoietin expression

RT-PCR was used to confirm whether erythropoietin-producing organoid precursor before transplantation into greater omentum and erythropoietin-producing organoid after transplantation into greater omentum express human erythropoietin mRNAs. Further, it was confirmed whether erythropoietin-producing organoid after transplantation into greater omentum specifically expressed human erythropoietin or not by double staining using anti-erythropoietin antibody and antihuman β₂ microglobulin antibody. The details of experiment are described as follows:

### - 1: Detection of mRNA using RT-PCR

Total RNA was extracted by RNeasy mini kit (QIAGEN GnbH, Hilden Germany) to synthesize cDNA using SuperScript II Reverse Transcriptase (Life Technologies BRL, Rockville, MD) according to the attached instruction protocol. Human erythropoietin (human EPO: 300bp), rat erythropoietin (rat EPO: 112bp), human microglobulin (hMG) and rat GAPDH (rGAPDH) were evaluated for their amplified products after PCR. Primer sequences and reaction conditions were as follows.

### Primer sequence:

Sense sequence of human microglobulin: caggtttact cacgtcatcc agc (SEQ ID NO: 1)
Antisense sequence of human microglobulin: tcacatggtt cacacggcag g (SEQ ID NO: 2)
Sense sequence of rat GAPDH: catcaacgac cccttcatt (SEQ ID NO: 3)
Antisense sequence of rat GAPDH: actccacgac atactcagca c (SEQ ID NO: 4)
Sense sequence of human erythropoietin: tacgtagcct cacttcactg ctt (SEQ ID NO: 5)
Antisense sequence of human erythropoietin: gcagaaagta tccgctgtga gtgttc (SEQ ID NO: 6)
Sense sequence of rat erythropoietin: tctgg gagcc cagaa ggaag ccat (SEQ ID NO: 7)
Antisense sequence of rat erythropoietin: ctgga gtgtc catgg gacag (SEQ ID NO: 8)

### Reaction conditions:

Conditions for human microglobulin and rat GAPDH: 94°C for 10 minutes, followed by 94°C for 1 minute, 43 cycles of 66°C and then 66°C for 10 minutes
Conditions for rat erythropoietin: 95°C for 15 minutes, followed by 95°C for 30 seconds, 55°C for 30 seconds, 40 cycles of 30 seconds at 72°C and then 72°C for 10 minutes
Conditions of human erythropoietin: 94°C for 10 minutes, followed by 94°C for 15 seconds, 60°C for 30 seconds, 30 cycles of 45 seconds at 72°C and then 72°C for 7 minutes

### - 2: Double staining using anti-erythropoietin antibody and antihuman β₂ microglobulin antibody

Paraffin-embedded tissues, immobilized with 10% formalin were sliced into 6 µm sections. The sections were deparaffinized with xylene and ethanol, followed by enhancing antigenicity with TUF (Target Unmasking Fluid, supplied by MONOSAN). The sections were reacted with two types of primary antibodies "goat antihuman EPO antibody: supplied by SANTA CRUZ (used in x 200) and mouse antihuman β₂ microglobulin antibody: supplied by PharMingen (used in x 400)" at 4°C overnight. Mouse IgG was detected by a signal amplification kit supplied by Molecular Probes (Alexa Fluor 568 Signal-Amplification kit for mouse antibody), while goat IgG was reacted with a biotinylated donkey anti-goat IgG antibody (supplied by R&D), followed by being colored using Tyramide Signal Amplification Kits supplied by Molecular Probe and observed under a fluorescence microscopy respectively.

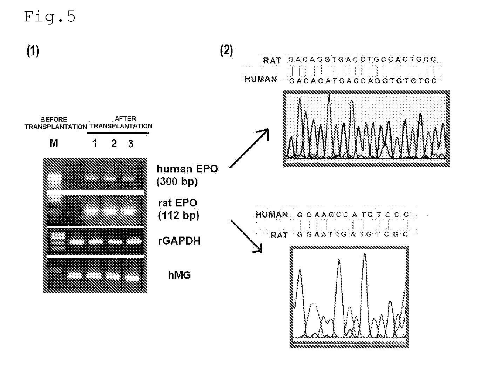

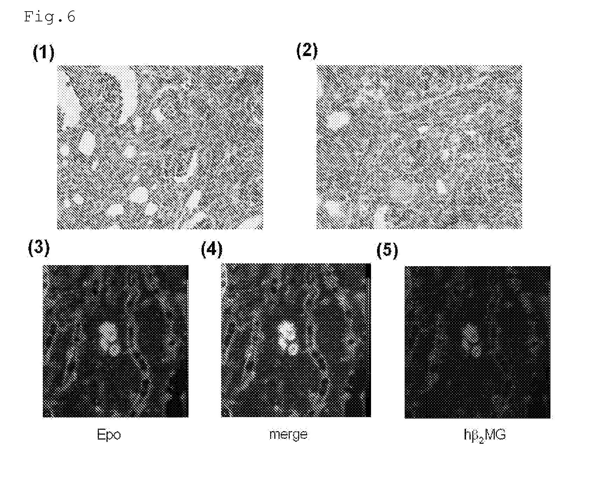

The results of RT-PCR are shown in Figure 5. It was confirmed from the results of Figure 5 that erythropoietin-producing organoid precursor before transplantation into greater omentum does not express erythropoietin mRNA, but erythropoietin-producing organoid after transplantation into greater omentum expresses erythropoietin mRNA (Figure 5(1)). Further, it was confirmed that this erythropoietin mRNA was a gene sequence derived from human (Figure 5(2)).
The results of double staining using anti-erythropoietin antibody and antihuman β₂ microglobulin antibody are shown in Figure 6. From the results of Figure 6, places where erythropoietin expresses were stained, and thus the expression of erythropoietin could be confirmed in an immunohistochemical examination (Figures 6(1) and (2)). Further, Figure 6 (3) shows that erythropoietin was stained at the same positions as those of human-derived microglobulin shown in Figure 6 (5) [Figure 6 (4) is an overlapping image of (3) and (5)]. Thus, it was confirmed that the erythropoietin-producing organoid after transplantation into greater omentum does not express erythropoietin derived from host (rat), but specifically expresses human erythropoietin mRNA.
The above results demonstrate that the transplanted human mesenchymal stem cell was differentiated into erythropoietin-producing organoid.

### 7) Examination of abilities of erythropoietin-producing organoid to produce erythropoietin and to adjust the production thereof

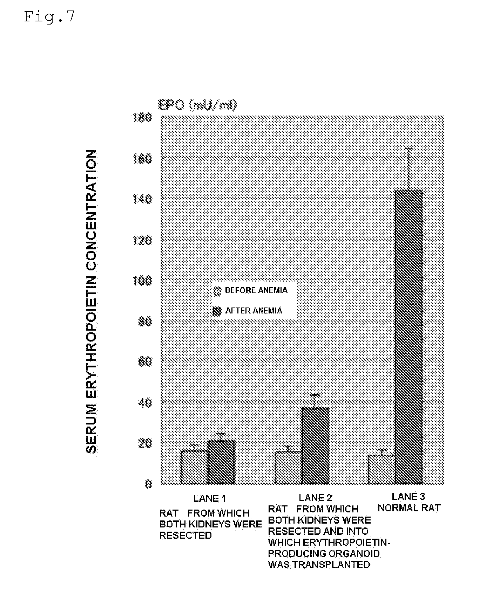

It was confirmed whether erythropoietin-producing organoid had abilities to produce erythropoietin and to adjust the production thereof or not. More specifically, rats received general anesthesia with Nembutal, then in order to cause anemia, blood equivalent to 2% of the body weight was rapidly hemorrhaged from the inferior vena cava, and the same amount of physiological saline solution was rapidly injected. Simultaneously, both kidneys were resected and the erythropoietin-producing organoid precursor obtained in 3) described above was transplanted into rat greater omentum (Lane 2 in Figure 7). Meanwhile, as a control, a rat from which both kidneys were removed (Lane 1 in Figure 7: erythropoietin-producing organoid precursor was not transplanted) and a normal rat (Lane 3 in Figure 7) were prepared. A fraction of blood was used in measuring erythropoietin. 24 hours later, the rats were anaesthetized again, the blood was collected by conducting cardiopuncture and the erythropoietin concentration was measured. The erythropoietin concentration was measured using RIA kit supplied by Mitsubishi Kagaku Iatron, Inc.. The erythropoietin concentration in the serum of each rat before and after anemia was measured by the method above.

Figure 7 shows the result of the measured erythropoietin concentration in the serum of each rat before and after anemia. The increase in erythropoietin was not observed in the rats after the resection of both kidneys (Lane 1 in Figure 7), while the significant increase in erythropoietin concentration was observed in the serum of rats having erythropoietin-producing organoid in greater omentum (Lane 2 in Figure 7). This demonstrates that the erythropoietin-producing organoid can produce erythropoietin. Further, no differences were observed in erythropoietin concentration in the serum before anemia among the rats having erythropoietin-producing organoid in greater omentum and the normal rat. This suggested that the erythropoietin-producing organoid should control (adjust) erythropoietin production in a normal state (not anemic state).

### 8) Examination of the effect of erythropoietin-producing organoid on correcting anemia

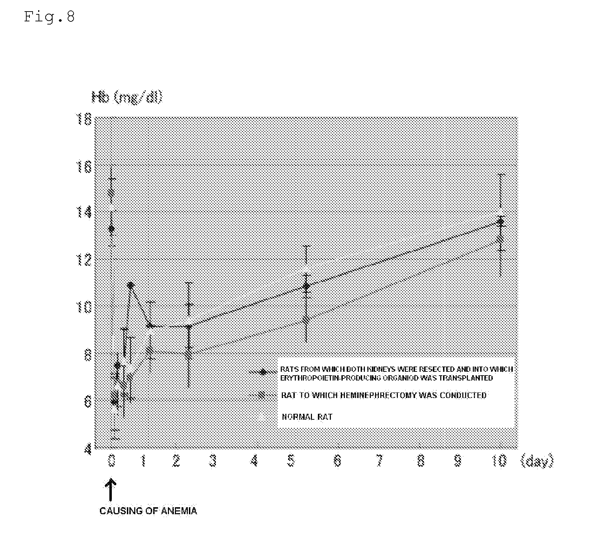

It was confirmed whether the erythropoietin produced by erythropoietin-producing organoid could actually affect bone marrow and correct anemia.
Rat models used in 7) above, from which both kidneys were resected died of renal failure in almost two days, and thus the degree of correction of anemia could not be confirmed. Thus rat models having a deteriorated kidney's ability to produce erythropoietin were used. The details are as follows.
An anti-glomerular basement membrane antibody was injected to rats to cause acute nephritis. Two weeks later, heminephrectomy was conducted (erythropoietin-producing organoid precursor was transplanted into greater omentum at this moment). Owing to that, kidney's ability to produce erythropoietin is significantly deteriorated, which delays the correction of anemia. Therefore, it was confirmed whether erythropoietin produced by erythropoietin-producing organoid could normalize this delay or not. Another two weeks later, the degree of correction of anemia was compared under the presence or absence of the erythropoietin-producing organoid by causing anemia by hemorrhage in the same manner as described in 7) above, collecting blood over time and measuring hemoglobin values in the blood. Meanwhile, Hb was measured using i-stat kit. Rats were treated as follows.
1. An anti-basement membrane antibody was administered to rats to cause nephritis, and then two weeks later, heminephrectomy was conducted (■ in Figure 8: erythropoietin-producing organoid precursor was not transplanted).
2. An anti-basement membrane antibody was administered to rats to cause nephritis, and then two weeks later, heminephrectomy was conducted (◆ in Figure 8: erythropoietin-producing organoid precursor was transplanted).
3. An anti-basement membrane antibody was administered to rats to cause nephritis. (▲ in Figure 8: no kidney was removed and erythropoietin-producing organoid precursor was not transplanted: normal rat).

The results of the effect of erythropoietin-producing organoid on correcting anemia were shown in Figure 8. It was shown that the correction of anemia was significantly delayed in the heminephrectomy rats (erythropoietin-producing organoid precursor was not transplanted) compared to the normal rat (■ in Figure 8). However, it was confirmed that rats having erythropoietin-producing organoid in greater omentum showed correction of anemia almost by taking the same course as that of the normal rat, though they received heminephrectomy (◆ in Figure 8). Thus it is suggested that erythropoietin produced from erythropoietin-producing organoid within greater omentum exerts an effect on correcting anemia.

From the results above, erythropoietin-producing organoid having the aforementioned feature can be produced from bone marrow-derived mesenchymal stem cell in the body of a patient (greater omentum), and the erythropoietin-producing organoid can exert an effect on correcting anemia.

### 9) Confirmation of the introduction of GDNF into mesenchymal stem cell by polymer

The possibility of controlled-release of GDNF was confirmed by mixing with a GDNF-containing polymer, instead of expressing GDNF in mesenchymal stem cell using replication-deficient recombinant adenovirus having human GDNF cDNA (AxCAhGDNF) described in Example 1.

### - 1: Confirmation of the controlled-release of GDNF using COS-1 cells

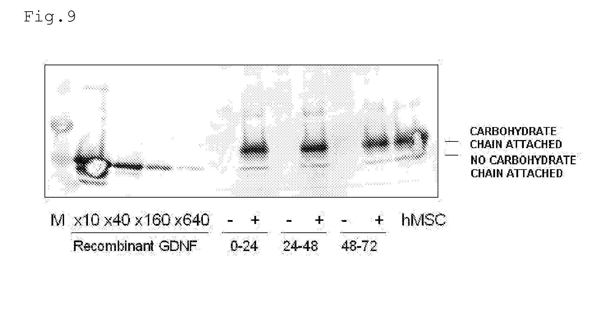

A culture supernatant in which COS-1 cells were infected with recombinant GDNF was dissolved in commercially available Mebiol Gel. Then, GDNF eluted for a period between 0 and 24 hours, between 24 and 48 hours and between 48 and 72 hours were examined by Western blot (Figure 9). In addition, "hMSCs" to the far right in Figure 9 is the control of culture supernatant from human mesenchymal stem cells into which GDNF was introduced using adenovirus.
In Figure 9, it could be observed that a sufficient amount of GDNF was eluted even for a period between 48 and 72 hours. It is shown from the above facts that GDNF dissolved in Mebiol Gel can be released under control.

### - 2: Confirmation of the controlled-release of GDNF in mesenchymal stem cells

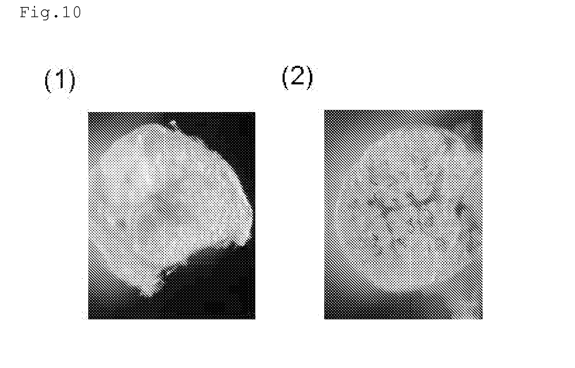

Human mesenchymal stem cells to which LacZ gene had been introduced was mixed with GDNF-containing Mebiol Gel (4°C), and injected into the nephrogenic site of rat embryo (E11.5), subjected to 24-hour whole-embryo culture and additional 6-day organ culture (Figure 10(1)). Meanwhile, as a control, human mesenchymal stem cells to which GDNF gene was introduced using adenovirus (no Figure) and those to which GDNF gene was not introduced (Figure 10(2)) were injected into the nephrogenic site of rat embryo (E11.5), subjected to 24-hour whole-embryo culture and additional 6-day organ culture. Then, LacZ positive parts were confirmed by X-Gal staining (Figure 10).
In Figure 10(1), a part derived from human mesenchymal stem cells was broadly stained by the controlled-release of GDNF gene (appeared blue in Figures), compared with human mesenchymal stem cells to which GDNF gene was not introduced (Figure 10(2)). Further, compared with human mesenchymal stem cells to which GDNF gene was introduced using adenovirus, the same degree of staining was observed in those parts (no Figure).
From the facts above, it was demonstrated that the controlled-release of GDNF can be obtained by GDNF-containing polymer, in particular by Mebiol Gel, without using adenovirus for gene introduction, and a part derived from human can be enlarged by mixing and injecting this gel and human mesenchymal stem cells.

### Industrial Applicability

The present invention can offer a new approach to a method for treating erythropoietin-related disorder, and for example, a recipient (patient), who receives an expensive recombinant protein, can produce an organoid carrying long sustainable abilities to produce erythropoietin and to adjust the production thereof by isolating autologous mesenchymal stem cells, transplanting this to a pregnant host animal and then transplanting into autologous greater omentum after being grown to a certain degree.

## Claims

1. A method for producing erythropoietin-producing organoid (organ-like structure) precursor, comprising the step of transplanting isolated mesenchymal stem cell derived from a mammal into an embryo within a pregnant mammalian host or an embryo separated from a pregnant mammalian host to thereby induce the differentiation of the mesenchymal stem cell, wherein a site to which the mesenchymal stem cell is to be transplanted is a nephrogenic site of the embryo, and a timing of transplantation corresponds to the stage in which a immune system of the host is still immunologically tolerant.

2. The method for producing erythropoietin-producing organoid precursor according to claim 1, further comprising in vitro whole-embryo culture.

3. The method for producing erythropoietin-producing organoid precursor according to claim 2, further comprising in vitro organ culture.

4. The method for producing erythropoietin-producing organoid precursor according to any one of claims 1 to 3, wherein the erythropoietin-producing organoid precursor has an ability to adjust erythropoietin production by transplantation into greater omentum of a mammal.

5. Erythropoietin-producing organoid precursor obtained by the method for producing erythropoietin-producing organoid precursor according to any one of claims 1 to 4.

6. Erythropoietin-producing organoid precursor obtained by the following steps:
(1) transplanting isolated mesenchymal stem cell derived from a mammal onto a nephrogenic site of an embryo separated from a pregnant mammalian host at a timing when the immune system of the host is still in the immunological tolerance stage, to thereby induce the differentiation of the mesenchymal stem cell,
(2) performing in vitro whole-embryo culture, and
(3) performing in vitro organ culture.

7. The erythropoietin-producing organoid precursor according to either of claims 5 or 6, wherein the erythropoietin-producing organoid precursor has an ability to adjust erythropoietin production by transplantation into greater omentum of a mammal.

8. A kit for producing erythropoietin-producing organoid precursor containing at least the following:
(1) isolated mesenchymal stem cell derived from a mammal,
(2) a pregnant mammal or an embryo separated from a pregnant mammal,
(3) a reagent for performing whole-embryo culture,
(4) a reagent for performing organ culture, and
(5) an instruction for producing erythropoietin-producing organoid precursor.

9. A method for treating erythropoietin-related disorder, comprising the step of transplanting the erythropoietin-producing organoid precursor according to any one of claims 5 to 7 into greater omentum of a mammal.

10. The method according to claim 9, wherein the erythropoietin-producing organoid precursor is derived from mesenchymal stem cell from a recipient (patient) with erythropoietin-related disorder.


SEQUENCE LISTING

<110>  Stem Cell Reserach Laboratory

<120>  Erythropoietin-producing organoid precursor

<130>  STEM-001-EP

<150>  JP 2006-185004
<151>  2006-07-04

<160>  8

<170>  PatentIn version 3.3

<210>  1
<211>  23
<212>  DNA
<213>  Sense primer for human MG

<400>  1
caggtttact cacgtcatcc agc                                              23


<210>  2
<211>  21
<212>  DNA
<213>  Antisense primer for human MG

<400>  2
tcacatggtt cacacggcag g                                                21


<210>  3
<211>  19
<212>  DNA
<213>  Sense primer for rat GAPDH

<400>  3
catcaacgac cccttcatt                                                   19


<210>  4
<211>  21
<212>  DNA
<213>  Antisense primer for rat GAPDH

<400>  4
actccacgac atactcagca c                                                21


<210>  5
<211>  23
<212>  DNA
<213>  Sense primer for human EPO

<400>  5
tacgtagcct cacttcactg ctt                                              23


<210>  6
<211>  26
<212>  DNA
<213>  Antisense primer for human EPO

<400>  6
gcagaaagta tccgctgtga gtgttc                                           26

<210>  7
<211>  24
<212>  DNA
<213>  Sense primer for rat EPO

<400>  7
tctgggagcc cagaaggaag ccat                                              24


<210>  8
<211>  20
<212>  DNA
<213>  Antisense primer for rat EPO

<400>  8
ctggagtgtc catgggacag                                                   20